# EUROPEAN PATENT APPLICATION

(11) **EP 1 997 460 A1**
(43) Date of publication of application: **03.12.2008**
(21) Application number: 07707812.9
(22) Date of filing: 31.01.2007
(51) Int. Cl.: A61F 7/10, A61F 7/08, B32B 1/06

(54) **COOLING OR HEATING SHEET**

(30) Priority: 23.03.2006 JP 2006081680
(71) Applicant: Ohshin MLP Co., Ltd, Echizen-shi Fukui, 9150052 (JP)
(72) Inventor: OTA, Keizo, Echizen-shi, Fukui 915-0052 (JP)
(74) Representative: Brykman, Georges
(86) International application number: PCT/JP2007/051626
(87) International publication number: WO 2007/108235

(57) **Abstract**

The object of this invention is to provide a cooling or warming sheet which is a sheet-like structure comprising an aqueous gel layer and covering layers that surround the gel layer, wherein the sheet can be used two or more times and shows a low-resilient property depending on physical properties of the aqueous gel layer.

A cooling or warming sheet is used, that comprises a fibrous layer a, an air-impermeable sheet layer a, an aqueous gel layer, and an air-impermeable sheet layer b in this order, wherein the aqueous gel layer is unexposed, and **characterized in that** the aqueous gel layer has a jelly strength of 200 to 3,000g when the jelly strength is determined under the following conditions:
(Conditions for determining jelly strength)
Measuring apparatus: Compression-type analyzer for determining physical properties
Atmospheric conditions for determination: 20 degrees celsius, 65%RH
Shape to be compressed and area: Circle having a diameter of 30mm, 7.065cm²
Quantity to be compressed (Amount of displacement): 2mm
Compression rate: 1mm/second
Size of test sample: 20cm x 30cm x 6mm (thickness).

## Description

### Technical Field

This invention relates to a cooling or warming sheet that can be used to cool down or warm up an object such as a human body and an animal.

### Background Art

Conventionally, cooling materials and cooling pillows have been known, in which a gel comprising a water-absorbable polymer and water as its main components is filled. They are used by freezing the gel before use. Thus, they show an extremely stiff touch during use, especially at the beginning of use.

A sheet-like material, e.g., "Netsusama-sheet (Trade name)" has also been known, wherein a gel comprising a water-absorbable polymer and water as its main components is exposed and the material is used by sticking it with the gel on a human body. In this material the cooling proceeds by using the phenomenon that heat of vaporization is taken away from the gel that has been made on a base material. For example, patent literature 1 discloses a sticking material that is a sheet-like, aqueous gel and that has no base material.

On the other hand, patent literature 2 discloses a cooling material e.g., a cooling pillow, using a water-permeable container, in which a gel comprising a water-absorbable polymer and water as its main components is filled. This cooling material can be used by freezing the gel before use, or, by expecting a cooling effect based on heat of vaporization without freezing. The water-permeable container of this cooling material is composed of a third-dimensional fiber structure, such as one that has been made by piling nonwoven fabrics and sewing the piled nonwoven fabrics. Therefore, even if the gel has been freezed, a suitable performance as a cushion can be attained by the third-dimensional fiber structure. Specifically, a head does not get to a bottom when the head is put on it, and the original shape can be quickly restored after the head was removed.

Patent literature 3 discloses a cooling or heating gelatinous pillow that has been made by filling an aqueous gel comprising a water-absorbable polymer, water, and an antifreeze agent into a flat bag and sealing the bag. Even if this pillow is placed in a freezer, the aqueous gel does not freeze. Thus, this pillow excellently fits to the head.

Further, patent literature 4 discloses a gel-filled sheet that has been made by filling an aqueous gel comprising a water-absorbable polymer, water, and a functional substance such as kaolin into a flat bag and sealing the bag. This sheet is used after cooling in a refrigerator or a freezer, or after heating with an electronic oven.

Patent literature 1: Japanese patent early-publication No. 2004-231567
Patent literature 2: Japanese patent early-publication No. 2005-118059
Patent literature 3: Japanese patent early-publication No. 2002-233442
Patent literature 4: Japanese patent early-publication No. 2002-325787

### Disclosure of Invention

### Problems to be Solved

The object of this invention is to provide a cooling or warming sheet which is a sheet-like structure comprising an aqueous gel layer and covering layers that cover the gel layer, wherein the sheet can be used two or more times and shows a low-resilient property depending on physical properties of the aqueous gel layer.

### Means for Solving Problems

The inventor has extremely studied to solve the above problems and has accomplished the following invention.

Namely, this invention relates to a cooling or warming sheet comprising a fibrous layer a, an air-impermeable sheet layer a, an aqueous gel layer, and an air-impermeable sheet layer b in this order, wherein the aqueous gel layer is unexposed, characterized in that the aqueous gel layer has a jelly strength of 200 to 3,000g when the jelly strength is determined under the following conditions:
(Conditions for determining jelly strength)
Measuring apparatus: Compression-type analyzer for determining physical properties
Atmospheric conditions for determination: 20 degrees celsius, 65%RH
Shape to be compressed and area: Circle having a diameter of 30mm, 7.065cm²
Quantity to be compressed (Amount of displacement): 2mm
Compression rate: 1mm/second
Size of test sample: 20cm x 30cm x 6mm (thickness).

The cooling or warming sheet of this invention may comprise any structural elements in addition to the above structural elements.

The cooling or warming sheet of this invention may comprise, in addition, a fibrous layer b on the outside of the air-impermeable sheet layer b.

The cooling or warming sheet of this invention may comprise, in addition, a fibrous layer c between the air-impermeable sheet layer a and the aqueous gel layer, and/or between the aqueous gel layer and the air-impermeable sheet layer b.

The cooling or warming sheet of this invention may comprise, in addition, a pressure-sensitive adhesive layer on the outside of the fibrous layer a, the fibrous layer b, or the air-impermeable sheet layer b.

The cooling or warming sheet of this invention may comprise, in addition, a bonding layer(s) for bonding a layer to another layer.

The amount of the water that the aqueous gel layer bears is preferably 60 to 95% on the basis of the weight of the aqueous gel layer.

The weight of the aqueous gel layer is preferably 1 to 15kg/m².

In the cooling or warming sheet of this invention, all elements of the sheet are preferably sewed together in four peripheries of the sheet, especially when the sheet is used for warming.

In the cooling or warming sheet of this invention, all elements of the sheet are preferably sewed together in any portion(s) other than four peripheries of the sheet, especially when the sheet has a laege size.

When the sheet is a pad to be laid, a sheet for a Japanese cushion, or a sheet for a back of a chair, the sheet is preferably sewed to give 2 to 30 divisions.

### Effects of Invention

According to this invention, a cooling or warming sheet that shows low-resilient property, in other words, that enables to disperse body pressure, is provided. When the cooling or warming sheet of this invention is used, a distress of human body is relieved and a person can spend comfortably because the sheet shows low-resilient property and enables to disperse body pressure.

The cooling or warming sheet of this invention shows a cooling effect without cooling in advance. However, it can be used after cooling in a refrigerator. By using this sheet, an appropriate cooling effect can be attained. Thus, sweet sleeping can be realized in, e.g., a summer season.

On the other hand, by using the cooling or warming sheet of this invention after warming up in an electronic oven, an appropriate warming effect can be attained. Thus, the use of this sheet that has been warmed up enables that, for example, a person who cannot sleep because of the chilliness of toes sleeps sweetly.

The cooling or warming sheet of this invention can be used in plural times by cooling in a refrigerator or by warming in an electronic oven.

### Brief Description of Drawings

[Figure 1] It is a cross-sectional view that shows one example of the cooling or warming sheet of this invention.
[Figure 2] It is a cross-sectional view that shows another example of the cooling or warming sheet of this invention.
[Figure 3] It is a cross-sectional view that shows still another example of the cooling or warming sheet of this invention.
[Figure 4] It is a cross-sectional view that shows still another example of the cooling or warming sheet of this invention.
[Figure 5] It is a schematic view that shows a method for determining a jelly strength of a gel.
[Figure 6] It is a schematic, plane view that shows one example of a sheet for warming up a sole.
[Figure 7] It is a schematic, plane view that shows one example of a pad to be laid.
[Figure 8] It is a schematic view that shows a method for determining a temperature of a gel.

### Description of Symbols

1, 9, 13, and 15 nonwoven fabric
2 thread
3, 7 air-impermeable polymer film
5 aqueous gel
10, 30 bag
17 pressure-sensitive adhesive layer
19 release sheet
100, 200, 300 sheet
400 sheet (a sheet for warming up a sole)
500 a pad to be laid

### Best Mode to Practice Invention

Below, this invention would be explained based on the best mode to practice the invention.

The cooling or warming sheet of this invention includes every sheet that satisfies the requirements of claim 1. Examples of the sheet include a sheet for cooling down a forehead, a cooling pillow, a sheet for a pillow, a pad to be laid, a sheet for a Japanese cushion, a sheet for a back of a chair, a sheet for a shoe, a mat for a pet, and a sheet for warming up a sole.

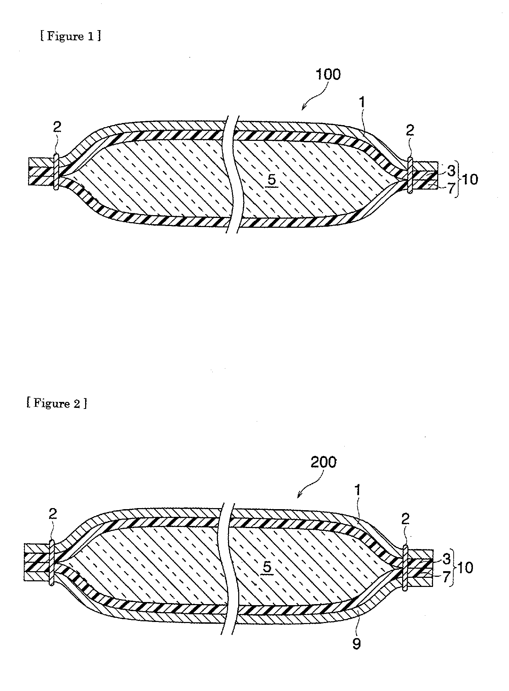

Next, the physical structures of the sheet of this invention would be explained referring to Figures 1 to 4 that show examples of the sheet.

Figure 1 is a cross-sectional view that shows one example of the sheet of this invention.

A sheet 100 is constituted of, from the top to the bottom and in the following order, a nonwoven fabric 1 as the fibrous layer a, an air-impermeable polymer film 3 as the air-impermeable sheet layer a, an aqueous gel 5, and an air-impermeable polymer film 7 as the air-impermeable sheet layer b. The nonwoven fabric 1 is entirely bonded to the air-impermeable polymer film 3. The air-impermeable polymer film 3 and the air-impermeable polymer film 5 form into a bag 10. In four peripheries of this sheet 100, sewing has been carried out with a thread 2.

Figure 2 is a cross-sectional view that shows another example of the sheet of this invention.

A sheet 200 is constituted of, from the top to the bottom and in the following order, a nonwoven fabric 1 as the fibrous layer a, an air-impermeable polymer film 3 as the air-impermeable sheet layer a, an aqueous gel 5, an air-impermeable polymer film 7 as the air-impermeable sheet layer b, and a nonwoven fabric 9 as the fibrous layer b. The nonwoven fabric 1 is entirely bonded to the air-impermeable polymer film 3. Similarly, the air-impermeable polymer film 7 is entirely bonded to the nonwoven fabric 9. The air-impermeable polymer film 3 and the air-impermeable polymer film 5 form into a bag 10. In four peripheries of this sheet 100, sewing has been carried out with a thread 2.

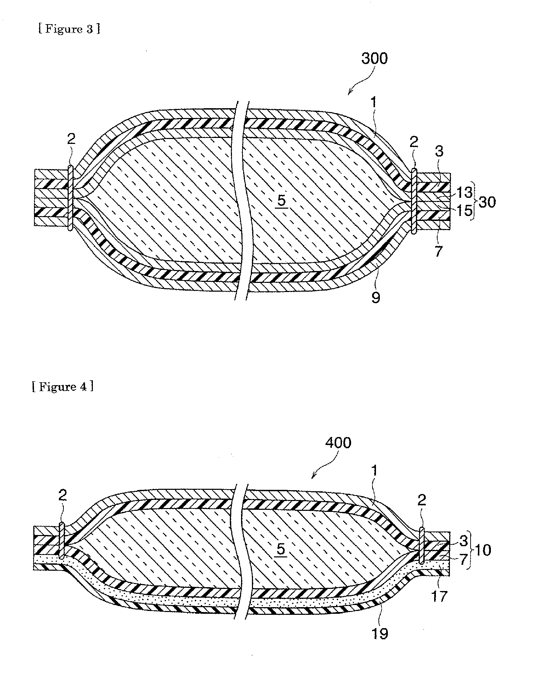

Figure 3 is a cross-sectional view that shows still another example of the sheet of this invention.

A sheet 300 is constituted of, from the top to the bottom and in the following order, a nonwoven fabric 1 as the fibrous layer a, an air-impermeable polymer film 3 as the air-impermeable sheet layer a, a nonwoven fabric 13 as the fibrous layer c, an aqueous gel 5, a nonwoven fabric 15 as the fibrous layer c, an air-impermeable polymer film 7 as the air-impermeable sheet layer b, and a nonwoven fabric 9 as the fibrous layer b. The nonwoven fabric 1, the air-impermeable polymer film 3, and the nonwoven fabric 13 are entirely bonded together. Similarly, the nonwoven fabric 15, the air-impermeable polymer film 7, and the nonwoven fabric 9 are entirely bonded together. The nonwoven fabric 13 and the nonwoven fabric 15 form into a bag 30. In four peripheries of this sheet 300, sewing has been carried out with a thread 2.

In the example that is shown in Figure 3, there is the nonwoven fabric 13 as the fibrous layer c between the air-impermeable polymer film 3 and the aqueous gel 5. Similarly, there is the nonwoven fabric 15 as the fibrous layer c between the air-impermeable polymer film 7 and the aqueous gel 5. The sheet of this invention may be an embodiment wherein there is only one of the nonwoven fabric 13 and the nonwoven fabric 15.

Because there is the fibrous layer c in the example that is shown in Figure 3, the heat transfer by conduction to an object (e.g., human skin) that is to be cooled or warmed is mildly carried out. Also, the aqueous gel layer adheres to the fibrous layer c, and is rarely shifted.

Figure 4 is a cross-sectional view that shows still another example of the sheet of this invention.

A sheet 400 is constituted of, from the top to the bottom and in the following order, a nonwoven fabric 1, an air-impermeable polymer film 3, an aqueous gel 5, an air-impermeable polymer film 7, a pressure-sensitive adhesive layer 17, and a release sheet 19. The air-impermeable polymer film 3 and the air-impermeable polymer film 7 form into a bag 10. In four peripheries of this sheet 400, sewing has been carried out with a thread 2.

This example corresponds to the example that is shown in Figure 1 wherein the pressure-sensitive adhesive layer 17 is made on the outside of the air-impermeable polymer film 7, and the surface of the pressure-sensitive adhesive layer 17 is covered with the release sheet 19.

In the example that is shown in Figure 2 or 3, the pressure-sensitive adhesive layer 17 may be made on the outside of the nonwoven fabric 1 or 9, and the surface of the pressure-sensitive adhesive layer 17 may be covered with the release sheet 19.

An outer peripheral portion, namely four peripheries, of the two layers (for example, the air-impermeable polymer film 3 and the air-impermeable polymer film 7 in the sheet 100 of Figure 1) that meet the aqueous gel may be bonded with an adhesive or may be heat-sealed.

Next, each element that constitutes the cooling or warming sheet of this invention would be specifically explained with examples.

The cooling or warming sheet of this invention comprises a fibrous layer a, an air-impermeable sheet layer a, an aqueous gel layer, and an air-impermeable sheet layer b in this order. It may also comprises a fibrous layer b on the outside of the air-impermeable sheet layer b. In addition, it may comprise a fibrous layer c between the air-impermeable sheet layer a and the aqueous gel layer, and/or, between the aqueous gel layer and the air-impermeable sheet layer b.

The fibrous layer that is used in the sheet of this invention is composed of, for example, at least one selected from the group consisting of a nonwoven fabric, a woven fabric, a nit, and paper. When the fibrous layer is made of the nonwoven fabric, its raw material is, for example, rayon, nylon, polyester, acrylic, polypropylene, vinylon, polyethylene, urethane, cotton, or cellulose, and its thickness is usually 20g/m² to 100g/m², preferably 30g/m² to 70g/m², and more preferably 40g/m² to 60g/m². When the fibrous layer is made of the woven fabric, its raw material is, for example, cotton, rayon, polyester, polypropylene, nylon, or acrylic, and its thickness is usually 50g/m² to 150g/m², preferably 60g/m² to 120g/m², and more preferably 70g/m² to 100g/m². When the fibrous layer is made of the nit, its raw material is, for example, rayon, polyester, polypropylene, nylon, or acrylic, and its thickness is usually 80g/m² to 200g/m², preferably 100g/m² to 180g/m², and more preferably 120g/m² to 150g/m².

The nonwoven fabric that is used for the fibrous layer includes a melt-blown nonwoven fabric, a spun-bonded nonwoven fabric, a spun-lace nonwoven fabric, and the like. In this invention every nonwoven fabric may be used.

A representative example of the air-impermeable sheet layer that is used in this invention is an air-impermeable polymer film. The thickness of the air-impermeable polymer film is usually 100µm or less, preferably 10 to 70µm, more preferably 20 to 50µm, and especially preferably 25 to 45µm.

Examples of the polymer that is a raw material of the air-impermeable sheet include polyethylene, polypropylene, polyester, polyamide, poly(vinyl chloride), poly(vinylidene chloride), polyurethane, polystyrene, ethylene-vinyl acetate copolymer, and polycarbonate.

The air-impermeable sheet is not restricted to a mono-layered film, and may be a multiple-layered film.

The aqueous gel layer is made by crosslinking a polymer that gelatinizes by absorbing smoothly a lot of water with a crosslinking agent under the presence of water and other components. Most preferable polymers that are used for the formation of the aqueous gel layer are polyacrylic acids. Examples of the polyacrylic acids include polyacrylic acid, a salt of polyacrylic acid, partially-neutralized polyacrylic acid (a copolymer of acrylic acid and a salt of acrylic acid), polymethacrylic acid, a salt of polymethacrylic acid, and partially-neutralized polymethacrylic acid (a copolymer of methacrylic acid and a salt of methacrylic acid). Specific examples of the salt include sodium salt, potassium salt, monoethanolamine salt, diethanolamine salt, triethanolamine salt, and ammonium salt. The amount of the polyacrylic acids is about 2 to 10% by weight based on the total amount of the gel.

In this invention, the aqueous gel layer is made by crosslinking the polymer that can gelatinize, preferably any polyacrylic acid, with, e.g., a multivalent metal. The multivalent metals include multivalent metals, salts of multivalent metals, and multivalent metallic compounds. Every multivalent metal that is di- or more valent and can crosslink any polyacrylic acid can be used.

Examples of the multivalent metal include multivalent metals such as magnesium, calcium, zinc, cadmium, aluminium, titanium, manganese, cobalt, and nickel, salts of them, and compounds of them. From the viewpoints of safety, productivity, and gel performances, aluminium, magnesium, calcium, and compounds comprising these metals are preferable, and aluminium compounds are especially preferable.

Examples of the aluminium compound include hydroxides such as aluminium hydroxide and aluminium magnesium hydroxide; normal salts and basic salts of inorganic and organic acids such as aluminium chloride, aluminium sulfate, dihydroxyaluminium aminoacetate, kaolin, and aluminium stearate; double salts such as aluminium alum; aluminates such as sodium aluminate, inorganic aluminium complex salts, and organic aluminium chelate compounds; synthetic hydrotalcite, magnesium metasilicate aluminate, aluminium nitrate, aluminium sulfate, EDTA-aluminium, aluminium allantoinate, aluminium acetate, and aluminium glycinal.

In this invention, to make the aqueous gel layer, a multivalent alcohol such as propylene glycol and a polymer other than polyacrylic acids may be used in addition to water, a polymer that can gelatinize (preferably any polyacrylic acid), and a crosslinking agent. By blending the polymer other than polyacrylic acids, too, the stability of the gel is improved.

Polymers other than polyacrylic acids include cellulose derivatives, starch derivatives, and vinyl polymers. Specific examples include cellulose derivatives such as carboxymethyl cellulose, hydroxymethyl cellulose, hydroxyethyl cellulose, hydroxymethylethyl cellulose, hydroxypropyl cellulose, hydroxypropyl cellulose phthalate methyl cellulose, cellulose acetate, cellulose acetate hydroxymethylethyl cellulose, methyl cellulose, ethyl cellulose, and alkali metal salts of them; starch derivatives such as carboxymethyl starch and hydroxypropyl starch; and vinyl polymers such as poly(vinyl alkyl ether), polyvinyl alcohol, polyvinyl acetate, methyl vinyl ether/maleic anhydride copolymer, polyvinyl pyrrolidone, carboxyvinyl polymer, vinyl pyrrolidone/vinyl acetate alkylaminoacrylic acid copolymer, styrene/maleic acid copolymer, ethylene/vinyl acetate copolymer, and vinyl acetate/polyvinylalcohol copolymer. Among them, cellulose derivatives are preferable, and carboxymethyl cellulose and its alkali metal salts are more preferable. The amount of the cellulose derivative or the like is about 2 to 10% by weight based on the total amount of the aqueous gel.

To make the aqueous gel layer, antiseptics, preservatives, stabilizers, coloring agents, pH regulators, agents for maintaining a shape, thickeners, and the like may be used in addition to the above components, unless the component affects the physical properties of the aqueous gel layer.

In the sheet of this invention, the aqueous gel layer is held in a bag that is made of other structural materials, for example, the air-impermeable sheet layer a and the air-impermeable sheet layer b, and is unexposed.

In this invention the aqueous gel layer has a jelly strength of 200 to 3,000g, preferably 500 to 2,000g, and more preferably 800 to 1,500g when determined under the following conditions:
(Conditions for determining jelly strength)
Measuring apparatus: Compression-type analyzer for determining physical properties
Atmospheric conditions for determination: 20 degrees celsius, 65%RH
Shape to be compressed and area: Circle having a diameter of 30mm, 7.065cm²
Quantity to be compressed (Amount of displacement): 2mm
Compression rate: 1mm/second
Size of test sample: 20cm x 30cm x 6mm (thickness).

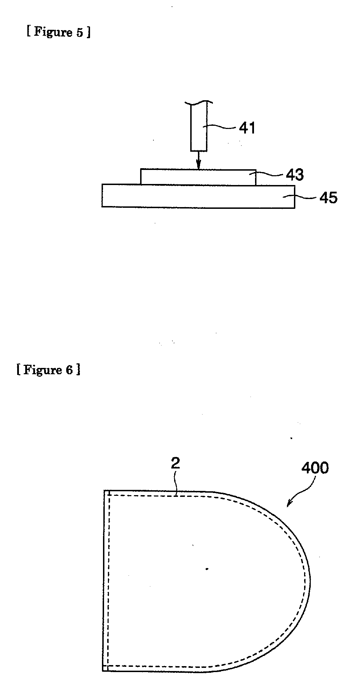

The outline for determining the jelly strength of the aqueous gel is shown in Figure 5. Namely, an adaptor (size and shape of the portion to be pressed: circle having a diameter of 30mm) 41 is pressed on a gel sample 43 that is placed on a sample rack 45 at a compression rate of 1 mm/second so as to be an amount of displacement of 2mm, and the load is determined at that time.

The jelly strength of the aqueous gel has been rising after the gel's formation with the progress of crosslinking of the polymer, and comes to a steady value after a lapse of a certain time. Therefore, the above value of the jelly strength should be satisfied, at least, when a consumer uses a product of this invention (usually five or more days after its production).

The aqueous gel of this invention is excellent in a shape-maintenance property, has the above jelly strength, and shows a low-resilient property because the polymer is crosslinked.

A method for determining a jelly strength is provided in JIS K6503-1996 for glue and gelatin. However, its conditions for determination differ from those that are adopted in the method of this invention.

The amount of water that the aqueous gel layer bears is preferably 60 to 95%, more preferably 70 to 90%, and especially preferably 75 to 85% on the basis of the weight of the aqueous gel layer.

The weight of the above aqueous gel layer is preferably 1 to 15 kg/m², more preferably 2 to 10kg/m², and especially preferably 3 to 6kg/m².

The cooling or warming sheet of this invention may comprise, in addition, a pressure-sensitive adhesive layer on the outside of the fibrous layer a, the fibrous layer b, or the air-impermeable sheet layer b.

Examples of the pressure-sensitive adhesive composition that is used to adhere to human body or cloth (e.g., underwear) include rubber-type pressure-sensitive adhesive compositions, acrylic pressure-sensitive adhesive compositions, and other pressure-sensitive adhesive compositions each comprising a thermoplastic resin (e.g., a polyamide, polyethylene, or cellulose resin) as a main component.

Examples of the pressure-sensitive adhesive that are used in the rubber-type pressure-sensitive adhesive compositions include diene-type polymeric compounds, specifically natural rubber, synthetic rubbers, and mixtures of them. Examples of the synthetic rubber include styrene-isoprene block copolymer, styrene-isoprene-styrene block copolymer, styrene-isobutylene-styrene block copolymer, styrene-butadiene rubber, polyisoprene rubber, butyl rubber, chloroprene rubber, nitrile rubber, polysulfide rubber, and silicone rubber.

Examples of the pressure-sensitive adhesive that are used in the acrylic pressure-sensitive adhesive compositions include conventionally used copolymers of at least one (meth)acrylate such as n-butyl (meth)acrylate, hexyl (meth)acrylate, decyl (meth)acrylate, dodecyl (meth)acrylate, or tridecyl (meth)acrylate, with a functional monomer (such as (meth)acrylic acid, maleic acid, maleic anhydride, hydroxyethyl acrylate, hydroxypropyl acrylate, acrylamide, dimethylacrylamide, aminoethyl methacrylate, or methoxyethyl (meth)acrylate) or a vinyl monomer (such asacrylonitrile, vinyl acetate or vinyl propionate) that is copolymerizable with the (meth)acrylate.

The physical condition of the pressure-sensitive adhesive composition that is used to form the pressure-sensitive adhesive layer is not particularly restricted insofar as the composition can be easily applied on a surface of an object, and is, for example, an emulsion, a solvent solution, an aqueous solution, or a hot melt type.

The thickness of the pressure-sensitive adhesive layer that is constituted of the pressure-sensitive adhesive composition is usually 10 to 70 µm and preferably 20 to 40µm.

The surface of the pressure-sensitive adhesive layer is covered with a release sheet. The raw materials for the release sheet are not restricted insofar as they have been conventionally used for a covering sheet for the pressure-sensitive adhesive layer. For example, various plastic films, metal foils, and laminates of plastic films and paper are used as the release sheet. On the release sheet, a coating agent for release of a silicone-, alkylacrylate-, or fluorine-type may be applied. Examples of the polymer as a raw material that constitutes the plastic film include polyethylene terephthalate, polyester, polypropylene, polyethylene, alkylbenzene sulfinates, and poly(vinyl chloride).

Next, processes for preparing the cooling or warming sheet of this invention would be explained.

The cooling or warming sheet 100 shown in Figure 1 is prepared, e.g., as follows.

A complex x is prepared by entirely or partially bonding a nonwoven fabric 1 that is to be a fibrous layer a to an air-impermeable polymer film 3 that is to be an air-impermeable sheet layer a with an adhesive or by thermal fusion bonding.

A composition for an aqueous gel is prepared by a usual method. The composition for an aqueous gel that has been prepared is applied on an air-impermeable polymer film 7 that is to be an air-impermeable sheet layer b to form an aqueous gel layer having a desirable thickness. It is preferable that the aqueous gel layer is not formed on portions that are to be four peripheries of the sheet 100 or on at least both sides of the film when the air-impermeable polymer film 7 is a long, rolled one.

The surface of the applied composition for an aqueous gel is covered with the above complex x at about the same time when the composition is applied. At this time, the surface of the air-impermeable polymer film 3 faces the gel.

In four peripheries of the sheet 100, sewing is carried out. At this time, not to expose the cut surfaces of the outer peripheries rolled seam or the like may be carried out.

When the cooling or warming sheet 200 shown in Figure 2 is prepared, a complex y of a nonwoven fabric 9 with an air-impermeable polymer film 7 may be used instead of the air-impermeable polymer film 7, and an aqueous gel layer may be prepared on the air-impermeable polymer film 7.

When the cooling or warming sheet 300 shown in Figure 3 is prepared, first a complex z is prepared by entirely or partially bonding a nonwoven fabric 1 that is to be a fibrous layer a, an air-impermeable polymer film 3 that is to be an air-impermeable sheet layer a, and a nonwoven fabric 13 that is to be a fibrous layer c together with an adhesive or by thermal fusion bonding. Similarly, a complex w is prepared by entirely or partially bonding a nonwoven fabric 9 that is to be a fibrous layer b, an air-impermeable polymer film 7 that is to be an air-impermeable sheet layer b, and a nonwoven fabric 15 that is to be a fibrous layer c together with an adhesive or by thermal fusion bonding.

On the nonwoven fabric 15 of the complex w, a composition for an aqueous gel layer is applied to form an aqueous gel layer having a desirable thickness. It is preferable that the aqueous gel layer is not formed on portions that are to be four peripheries of the sheet 300 or on at least both sides of the complex w when the complex w is a long, rolled one.

The surface of the applied composition for an aqueous gel is covered with the above complex z at about the same time when the composition is applied. At this time, the surface of the nonwoven fabric 13 faces the gel.

In four peripheries of the sheet 300, sewing is carried out.

The cooling or warming sheet 400 shown in Figure 4 is prepared as follows.

First, the cooling or warming sheet shown in Figure 1 is prepared by the method described above. Separately, on one surface of a release paper a pressure-sensitive adhesive layer 17 is prepared. The pressure-sensitive adhesive layer 17 is prepared by using a coating or printing machine such as an added gravure-printing type or a screen-printing type. At about the same time of the preparation of the pressure-sensitive adhesive layer 17, the surface of the layer 17 is coved with a release sheet 19.

To the air-impermeable polymer film of the cooling or warming sheet shown in Figure 1, the pressure-sensitive adhesive layer 17 is adhered while peeling the release paper.

The cooling or warming sheet having a pressure-sensitive adhesive layer 17 of this type has a use as a sheet 400 for warming up a sole of which plane view is shown in Figure 6 and a use as a warming sheet for a shoe that is used by inserting it into the shoe. The sheet 400 for warming up a sole is used by sticking it on outside or inside of the bottom (sole) of a sock.

The cooling or warming sheet of this invention is preferably a sheet wherein all elements of the sheet are sewed together in four peripheries of the sheet, especially when the sheet is used for warming. This is because the rupture of the sheet is prevented by escaping water vapor from the seams of threat when the sheet is warmed up with an electronic oven.

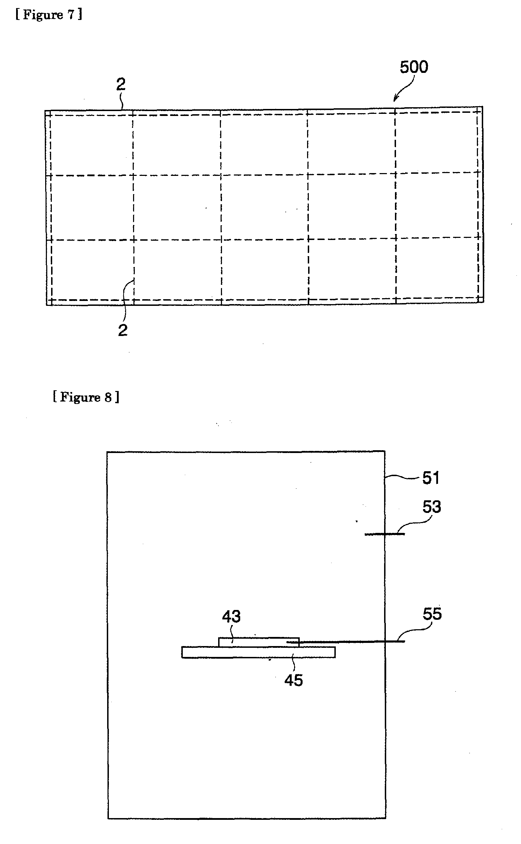

The cooling or warming sheet of this invention is preferably a sheet wherein all elements of the sheet are sewed together in any portion(s) other than four peripheries of the sheet, especially when the sheet has a big size. By that sewing the aqueous gel layer is steadily firmed. For example, in the pad 500 to be laid of which plane view is shown in Figure 7, the pad 500 is sewed to give 15 divisions.

In, e.g., a pad to be laid, a sheet for a Japanese cushion, or a sheet for a back of a chair, it is preferable that all elements of the sheet are sewed together to give 2 to 30 divisions

In the preparation of the cooling or warming sheet of this invention, the aqueous gel layer can also be prepared by casting, instead of applying a composition for an aqueous gel layer. For example, the sheet 200 that is shown in Figure 2 (with the proviso that the sheet is not sewn with the thread 2) can also be prepared as follows.

The above complex x (a long, rolled one) and the above complex y (a long, rolled one) are set so that the air-impermeable polymer film 3 faces the air-impermeable polymer film 7, and then heat-sealed or bonded with an adhesive to each other in their transverse direction. Next, the both side of the long, rolled ones are heat-sealed or bonded with an adhesive to each other to form a bag portion. A composition for an aqueous gel layer is casted into the bag portion. The bag portion is pressed to be a sheet-like shape, and then the long, rolled ones are heat-sealed or bonded with an adhesive to each other in their transverse direction. At about the center of the longitudinal direction of the portion that was formed latterly by heat-sealing or bonding the long, rolled ones to each other in their transverse direction, cutting is done. Thus, one cooling or warming sheet of this invention is given. Then, the cooling or warming sheet of this invention is continuously prepared in the same manner.

Below, this invention would be specifically explained by referring to its examples.

### Example 1

### (1) Determination of Jelly Strength of Gel

Test samples for determining jelly strengths of gels were prepared as follows, and their jelly strengths were determined.

(a) Material Constituting Each Layer
(Fibrous layer a) Spun-lace nonwoven fabric made of a polyester; basis weight: 45g/m²
(Air-impermeable sheet layer a) Polyethylene film; thickness: 40µm
(Aqueous gel layer) Prepared by using compositions of which formulas are shown in Table 1; thickness: 6mm
(Air-impermeable sheet layer b) Polyethylene film; thickness: 40µm
(Fibrous layer b) Spun-lace nonwoven fabric made of a polyester; basis weight: 45g/m²

The spun-lace nonwoven fabric made of a polyester was entirely bonded to the polyethylene film with an adhesive.

(b) Preparation Method
(i) On the polyethylene film of a base material that had been made by bonding the spun-lace nonwoven fabric made of a polyester (fibrous layer b) to the polyethylene film (air-impermeable sheet layer b), each of the compositions (Invention Examples 1 to 3) of which formulas are shown in Table 1 was applied so as to have a thickness of 6 mm.

(ii) At about the same time of the application of the composition, the surface of the gel was covered with an overlap material that had been made by bonding the spun-lace nonwoven fabric made of a polyester (fibrous layer a) to the polyethylene film (air-impermeable sheet layer a), wherein the polyethylene film faced the gel. Then, in four peripheries sewing was done.

(iii) The test samples (20cm x 30cm x 6mm) as prepared above were left in a thermostatic chamber of 40 degrees celsius for 2 days so that the polymers in the aqueous gel layers were acceleratingly crosslinked. Then, the test samples were left in a thermostatic chamber of 20 degrees celsius.

| Names of Raw Materials | Blended Amount ( weight % ) | | |
|---|---|---|---|
| | Invention Example 1 | Invention Example 2 | Invention Example 3 |
| Carboxymethyl Cellulose Sodium Salt | 3.0 | 3.0 | 3.0 |
| Polyacrylic Acid Partial Sodium Salt | 3.5 | 3.5 | 3.5 |
| Tartaric Acid | 0.3 | 0.3 | 0.3 |
| Propylene Glycol | 0.1 | 0.1 | 0.1 |
| Butyl Benzoate | 0.1 | 0.1 | 0.1 |
| Methyl Benzoate | 0.15 | 0.15 | 0.15 |
| Aluminium Hydroxide | 0.00 | 0.07 | 0.10 |
| Magnesium Metasilicate Aluminate | 0.075 | 0.075 | 0.075 |
| Disodi um Ethylenediaminetetraacetate | 0.040 | 0.040 | 0.040 |
| Glycerol | 8.5 | 8.5 | 8.5 |
| Food Dye Blue No. 1 | 0.0005 | 0.0005 | 0.0005 |
| Water | balance | balance | balance |
| Total | 100.0 | 100.0 | 100.0 |

### (c) Determination of Jelly Strength

On 14 days and 30 days after the applications of the compositions, the overlap materials were peeled off. Then, by using a simple-type analyzer for determining physical properties Leotex SD-700DP (manufactured by Sun Chemical Co., Ltd), the jelly strengths of the gels were determined under the following conditions. The outline of the determination is as shown in Figure 5. Namely, the an adaptor 41 was pressed on the gel sample 43 that is placed on the sample rack 45 and the load was determined at that time.

Three gel samples were determined for each of the Inventions examples 1 to 3.

Table 2 shows their results (minimum values, maximun values, and average values).

(Conditions for Determination)
Atmospheric conditions for determination: 20 degrees celsius, 65%RH
Shape to be compressed and area: Circle having a diameter of 30 mm, 7.065 cm²
Quantity to be compressed (Amount of displacement): 2 mm
Compression rate: 1 mm/second

| | 14 days after gel layer was made | | | 30 days after gel layer was made | | |
|---|---|---|---|---|---|---|
| | Minimum Value | Maximum Value | Average Value | Minimum Value | Maximum Value | Average Value |
| Invention Example 1 | 366.7 g | 568.6 g | 480.4 g | 430.8 g | 573.2 g | 489.7 g |
| Invention Example 2 | 530.5 g | 929.1 g | 682.1 g | 653.1 g | 1014.2 g | 803.4 g |
| Invention Example 3 | 845.0 g | 1080.0 g | 926.1 g | 971.2 g | 1288.0 g | 1093.7 g |

(2) Preparation of Pads To Be Laid and Sensory Test Three kinds of the pads to be laid each having the structure shown in Figure 2 were prepared.

(a) Material Constituting Each Layer
(Fibrous layer a) Spun-lace nonwoven fabric made of a polyester; basis weight: 45g/m²
(Air-impermeable sheet layer a) Polyethylene film; thickness: 40µm
(Aqueous gel layer) Prepared by using the composition of which formula is shown in Table 1 as Invention Example 2; applied amounts: 3kg/m², 5kg/m², and 7kg/m²
(Air-impermeable sheet layer b) Polyethylene film; thickness: 40µm
(Fibrous layer b) Spun-lace nonwoven fabric made of a polyester; basis weight: 45g/m²

The Spun-lace nonwoven fabric made of a polyester was entirely bonded to the polyethylene film with an adhesive.

(b) Preparation Method
(i) On the polyethylene film of a base material that had been made by bonding the spun-lace nonwoven fabric made of a polyester (fibrous layer b) to the polyethylene film (air-impermeable sheet layer b), the composition of Invention Example 2 of which formula is shown in Table 1 was applied in an applied amount of 3kg/m², 5kg/m², or 7kg/m².

(ii) At about the same time of the application of the composition, the surface of the gel was covered with an overlap material that had been made by bonding the spun-lace nonwoven fabric made of a polyester (fibrous layer a) to the polyethylene film (air-impermeable sheet layer a), wherein the polyethylene film faced the gel. Then, in four peripheries sewing was done.

Thus, pads to be laids each having a width of 92 cm and a length of 180 cm were prepared.

### (c) Sensory Test

The pads to be laid were left in a thermostatic chamber of 40 degrees celsius for 2 days so that the polymers in the aqueous gel layers were acceleratingly crosslinked. Then, the pads were left in a thermostatic chamber of 15 degrees celsius for 10 days.

In a room of which temperature was set at 30 degrees celsius, futons were laid, the pads were laid on the futons, and then they were covered with sheets of cotton broadcloth.

As a control, a futon on which no pad had been laid was also prepared.

By using a surface thermometer, surface temperatures of the sheets were determined.

The subjects lay on the sheets and regularly evaluated whether they felt coolly or warmly at neck, shoulders, loin, hip, legs and feet, and chest.

Table 3 shows the results.

| Time Lapsed (minutes) | | | 1 | 5 | 10 | 20 | 30 | 40 | 50 | 60 | 90 | 120 | 150 | 180 |
|---|---|---|---|---|---|---|---|---|---|---|---|---|---|---|
| Used a Pad To Be Laid | Gel: 3kg/m² Surface Temperature at the Start of the Experiment: 30.9°C | Neck | B | A | A | A | A | A | A | C | C | C | C | C |
| | | Shoulders | A | A | A | A | A | A | A | A | B | C | C | C |
| | | Loin | A | A | A | A | A | A | A | A | C | C | C | C |
| | | Hip | B | A | A | A | A | A | A | A | C | C | C | C |
| | | Legs/Feet | B | B | B | A | A | A | A | B | C | C | C | C |
| | | Arms | A | A | A | A | A | A | A | B | B | C | C | C |
| | Gel: 5kg/m² Surface Temperature at the Start of the Experiment: 30.8°C | Neck | B | A | A | A | A | A | A | A | C | C | C | C |
| | | Shoulders | A | A | A | A | A | A | A | A | A | B | C | C |
| | | Loin | A | A | A | A | A | A | A | A | A | C | C | C |
| | | Hip | B | A | A | A | A | A | A | A | A | A | C | C |
| | | Legs/Feet | B | B | B | A | A | A | A | A | B | C | C | C |
| | | Arms | A | A | A | A | A | A | A | A | B | B | C | C |
| | Gel 7kg/m² Surface Temperature at the Start of the Experiment: 31.1°C | Neck | B | A | A | A | A | A | A | A | A | A | C | C |
| | | Shoulders | A | A | A | A | A | A | A | A | A | A | A | B |
| | | Loin | A | A | A | A | A | A | A | A | A | A | A | C |
| | | Hip | B | A | A | A | A | A | A | A | A | A | A | C |
| | | Legs/Feet | B | B | B | A | A | A | A | A | A | A | B | C |
| | | Arms | A | A | A | A | A | A | A | A | A | A | B | B |
| Used No Pad (Futon Only) Surface Temperature at the Start of the Experiment: 30.9°C | | Neck | B | C | C | C | C | C | C | C | C | C | C | C |
| | | Shoulders | C | C | C | C | C | C | C | C | C | C | C | C |
| | | Loin | B | C | C | C | C | C | C | C | C | C | C | C |
| | | Hip | C | B | C | C | C | C | C | C | C | C | C | C |
| | | Legs/Feet | B | B | C | C | C | C | C | C | C | C | C | C |
| | | Arms | B | B | B | B | B | C | C | C | C | C | C | C |

| | | | | | | | | | | | | | | |
|---|---|---|---|---|---|---|---|---|---|---|---|---|---|---|
| (Note) A: Felt Coolly; B: Not Felt Coolly or Warmly; C: Felt Warmly | | | | | | | | | | | | | | |

As is clear from Table 3, when the pad was not used, the subject felt warmly at five portions other than arms 10 minutes after the start of the test and at all evaluated portions 40 minutes after the start of the test. When the pads were laid, the subjects felt warmly at all evaluated portions 120 minutes after the start of the test (applied amount: 3kg/m²) and 150 minutes after the start of the test (applied amount: 5kg/m²). In the case of the applied amount of 7kg/m², the subject felt warmly at only four portions 180 minutes after the start of the test.

The subject who used the futon on which no pad had been laid reported the lumbago depending on the sinking of the loin about 30 minutes after the start of the test. On the other hand, the subjects who used the futons on which the pads had been laid reported no lumbago and evaluated comfortable.

As shown above, by using the pad to be laid of this invention, comfortable sleep can be attained even if the temperature is high.

### (3) Determination of Temperature-Rising Property of Gel

### (a) Preparation of gel sample

The pads to be laid that had been prepared in the paragraph (2) were left in a thermostatic chamber of 40 degrees celsius for 2 days so that the polymers in the aqueous gel layers were acceleratingly crosslinked. Then, the pads were left in a thermostatic chamber of 10 degrees celsius for 10 days.

The gels in the pads to be laid that had been cooled were cut so as to have a size of 20cm x 30cm.

### (b) Determination of Temperature of Gel Sample

The gel samples that had been prepared in the paragraph (a) were placed in a thermostatic chamber of a hot-air circulation type, of which temperature had been set at 30 degrees celsius. The internal temperature of the gel samples were determined with a chromel-alumel temperature detector. Figure 8 shows schematically the situation when the temperature is determined. Namely, the gel sample 43 was placed in the thermostatic chamber 51 of a hot-air circulation type, and then the temperature in the thermostatic chamber 51 was determined with the chromel-alumel temperature detector 53 and the temperature of the inside (nearly the center of the gel in its thickness) of the gel sample was determined with the chromel-alumel temperature detector 55. Table 4 shows the results.

**Table 4 (No.1) (Unit : °C)**

| Time Lapsed (minutes) | | 0 | 10 | | 20 30 | 40 | 50 | 60 | 90 | 120 | 150 |
|---|---|---|---|---|---|---|---|---|---|---|---|
| Temperature in Thermostatic Chamber | | 28.4 | 29.0 | 29.1 | 29.1 | 29.2 | 29.3 | 29.3 | 29.4 | 29.4 | 29.4 |
| Temperature of Gel | Gel: 3kg/m² | 9.6 | 12.2 | 15.0 | 17.5 | 20.7 | 22.2 | 24.3 | 27. 5 | 28.6 | 29.4 |
| | Gel: 5kg/m² | 9.8 | 11.8 | 14.2 | 16.5 | 19.1 | 20.3 | 21.7 | 25.8 | 26.9 | 27.9 |
| | Gel: 7kg/m² | 9.8 | 11.7 | 13.8 | 15.3 | 17.0 | 18.7 | 20.0 | 21.3 | 25.5 | 26.8 |

**Table 4 (No. 2) (Unit : °C)**

| Time Lapsed (minutes) | | 180 | 210 | 240 | 270 | 300 | 330 | 360 | 390 | 420 |
|---|---|---|---|---|---|---|---|---|---|---|
| Temperature in Thermostatic Chamber | | 29.5 | 29.5 | 29.5 | 29.6 | 29.6 | 29.6 | 29.6 | 29.7 | 29.8 |
| Temperature of Gel | Gel: 3kg/m² | 29.5 | 29.4 | 29.6 | 29.5 | 29.6 | 29.5 | 29.7 | 29.8 | 29.7 |
| | Gel: 5kg/m² | 28.7 | 29.4 | 29.5 | 29.6 | 29.5 | 29.6 | 29.8 | 29.7 | 29.7 |
| | Gel: 7kg/m² | 27.9 | 28.6 | 28.9 | 29.1 | 29.2 | 29.5 | 29.4 | 29.8 | 29.7 |

As is clear from Table 4, the temperature-rising velocity was slow when there was a lot of gel. Especially, when the applied amount of the gel was 7 kg/m², it was needed for four hours or more to arrive 29 degrees celsius that was nearly the same as the temperature in the thermostatic chamber.

Also from this test result, it comes to be clear that by using the pad to be laid of this invention, comfortable sleep can be attained even if the temperature is high.

### Example 2

### (1) Preparation of Sheet for Warming Up Sole

A sheet for warming up a sole was prepared, of which structure was showed in Figures 4 and 6.

(a) Material Constituting Each Layer
(Fibrous layer a) Spun-lace nonwoven fabric made of a polyester; basis weight: 45g/m²
(Air-impermeable sheet layer a) Polyethylene film; thickness: 40µm
(Aqueous gel layer) Prepared by using the composition of which formula is shown in Table 1 as Invention Example 2; applied amounts: 3kg/m²
(Air-impermeable sheet layer b) Polyethylene film; thickness: 40µm
(Pressure-sensitive adhesive layer) Prepared by using a rubber-type, pressure-sensitive adhesive of which formula is shown in Table 5; thickness: 20µm
(Release sheet) Film made of a polyethylene terephthalate that has been processed with a silicone; thickness: 30µm

The spun-lace nonwoven fabric made of a polyester was entirely bonded to the polyethylene film with an adhesive.

**[Table 5]**

| Names of Raw Materials | Blended Amount (weight %) |
|---|---|
| Styrene· Isoprene· Styrene Block Copolymer | 37.0 |
| Aliphatic Saturated Hydrocarbon Resin | 30.0 |
| Paraffin-type Oil | 32.0 |
| Phenolic Antioxidant | 1.0 |
| Total | 100.0 |

(b) Preparation Method
(i) On one surface of a release paper, the rubber-type, pressure-sensitive adhesive of which formula is shown in Table 5 was applied to form a pressure-sensitive adhesive layer having a thickness of 20 µm. The surface of the adhesive layer was covered with a release sheet.

(ii) An overlap material was prepared wherein the spun-lace nonwoven fabric made of a polyester (fibrous layer a) was bonded to the polyethylene film (air-impermeable sheet layer a).

(iii) On one surface of the polyethylene film (air-impermeable sheet layer b), the composition of Invention Example 2 of which formula is shown in Table 1 was applied in an applied amount of 3kg/m².

(iv) At about the same time of the application of the composition, the surface of the gel was covered with the overlap material that had been made by bonding the spun-lace nonwoven fabric made of a polyester (fibrous layer a) to the polyethylene film (air-impermeable sheet layer a), wherein the polyethylene film faced the gel. Then, in four peripheries sewing was done.

(v) Onto the surface of the polyethylene film (air-impermeable sheet layer b), the pressure-sensitive adhesive layer that had been prepared in paragraph (i) was adhered while peeling off the release paper.

(vi) Thus, a sheet for warming up a sole was prepared, which has a shape shown in Figure 6 wherein its maximum width and length are 8 cm and 10 cm, respectively.

### (c) Determination of Jelly Strength

The jelly strength of the aqueous gel layer was determined 5 days after the preparation. The strength was about 300g.

### (d) Sensory Test

Sheets for warming up soles were warmed by using an electronic oven of 500W.
The release sheets were peeled off, the pressure-sensitive adhesive layers were stuck on the bottoms (backs) of socks, and the subject slept. The subject could comfortably sleep without chilliness of toes.

The subject was a woman of forties who suffered from chilliness of toes.

### Example 3

### Preparation of Pad To Be Laid and Sensory Test

The pad to be laid having the structure shown in Figure 3 was prepared. But, as the fibrous layers a and b, cotton broadcloth was used instead of the nonwoven fabric.

(a) Material Constituting Each Layer
(Fibrous layer a) 100 % Cotton Broadcloth manufactured by Nisshinbo Industries, Inc.; product No. 4400; yarn fineness: 40 counts x 40 counts; yarn density: 65 x 125
(Air-impermeable sheet layer a) Polyethylene film; thickness: 40µm
(Fibrous layer c) Spun-lace nonwoven fabric made of a polyester; basis weight: 45g/m²
(Aqueous gel layer) Prepared by using the composition of which formula is shown in Table 1 as Invention Example 2; applied amounts: 5kg/m²
(Fibrous layer c) Spun-lace nonwoven fabric made of a polyester; basis weight: 45g/m²
(Air-impermeable sheet layer b) Polyethylene film; thickness: 40µm
(Fibrous layer b) 100 % Cotton Broadcloth manufactured by Nisshinbo Industries, Inc.; product No. 4400; yarn fineness: 40 counts x 40 counts; yarn density: 65 x 125

The cotton broadcloth, the polyethylene film, and the spun-lace nonwoven fabric made of a polyester were entirely bonded together with an adhesive.

(b) Preparation Method
(i) On the spun-lace nonwoven fabric of a base material that had been made by bonding the cotton broadcloth (fibrous layer b), the polyethylene film (air-impermeable sheet layer b), and the spun-lace nonwoven fabric made of a polyester (fibrous layer c) together, the composition of Invention Example 2 of which formula is shown in Table 1 was applied in an applied amount of 5kg/m².

(ii) At about the same time of the application of the composition, the surface of the gel was covered with an overlap material that had been made by bonding the cotton broadcloth (fibrous layer a), the polyethylene film (air-impermeable sheet layer a), and the spun-lace nonwoven fabric made of a polyester (fibrous layer c) together, wherein the nonwoven fabric faced the gel. Then, sewing was done as shown in Figure 3. In four peripheries, rolled seam was done.

(iii) Thus, a pad to be laid having a width of 92 cm and a length of 180 cm was prepared

(iv) The pad to be laid was left in a thermostatic chamber of 40 degrees celsius for 2 days so that the polymers in the aqueous gel layer were acceleratingly crosslinked.

### (c) Sensory Test

In a room of which temperature was set at 30 degrees celsius, a futon was laid, the pad that had been kept under an ordinary temperature (about 25 degrees celsius) after preparation was laid on the futon, and then they were covered with a sheet of cotton broadcloth.

A subject lay on the sheet and slept for the night with a toweling blanket that was put.

The next day, in the room of which temperature was set at 30 degrees celsius, a futon was laid and the futon was covered with a sheet of cotton broadcloth, without using the pad to be laid. The subject slept for the night with the toweling blanket that was put.

When the pad to be laid was used, the subject could sleep until the next morning without waking up in the night. However, when the pad to be laid was not used, the subject could not sleep well and waked up several times.

## Claims

1. A cooling or warming sheet comprising a fibrous layer a, an air-impermeable sheet layer a, an aqueous gel layer, and an air-impermeable sheet layer b in this order, wherein the aqueous gel layer is unexposed, **characterized in that** the aqueous gel layer has a jelly strength of 200 to 3,000g when the jelly strength is determined under the following conditions:
(Conditions for determining jelly strength)
Measuring apparatus: Compression-type analyzer for determining physical properties
Atmospheric conditions for determination: 20 degrees celsius, 65%RH
Shape to be compressed and area: Circle having a diameter of 30mm, 7.065cm²
Quantity to be compressed (Amount of displacement): 2mm
Compression rate: 1mm/second
Size of test sample: 20cm x 30cm x 6mm (thickness).

2. The cooling or warming sheet as disclosed in claim 1,which comprises, in addition, a fibrous layer b on the outside of the air-impermeable sheet layer b.

3. The cooling or warming sheet as disclosed in claim 1 or 2, which comprises, in addition, a fibrous layer c between the air-impermeable sheet layer a and the aqueous gel layer, and/or between the aqueous gel layer and the air-impermeable sheet layer b.

4. The cooling or warming sheet as disclosed in any one of claims 1 to 3, which comprises, in addition, a pressure-sensitive adhesive layer on the outside of the fibrous layer a, the fibrous layer b, or the air-impermeable sheet layer b.

5. The cooling or warming sheet as disclosed in any one of claims 1 to 4, wherein the amount of the water that the aqueous gel layer bears is 60 to 95% on the basis of the weight of the aqueous gel layer.

6. The cooling or warming sheet as disclosed in any one of claims 1 to 5, wherein the weight of the aqueous gel layer is 1 to 15kg/m².

7. The cooling or warming sheet as disclosed in any one of claims 1 to 6, wherein all elements of the sheet are sewed together in four peripheries of the sheet.

8. The cooling or warming sheet as disclosed in any one of claims 1 to 7, wherein all elements of the sheet are sewed together in any portion(s) other than four peripheries of the sheet.

9. The cooling or warming sheet as disclosed in claim 8, wherein the sheet is a pad to be laid, a sheet for a Japanese cushion, or a sheet for a back of a chair, and the sheet is sewed to give 2 to 30 divisions.
